# EUROPÄISCHE PATENTSCHRIFT

(11) **EP 2 092 279 B1**
(45) Veröffentlichungstag und Bekanntmachung des Hinweises auf die Patenterteilung: **16.01.2019**
(21) Anmeldenummer: 07847590.2
(22) Anmeldetag: 30.11.2007
(51) Int. Cl.: G01F 23/24, G01F 23/26

(54) **VORRICHTUNG ZUR BESTIMMUNG UND/ODER ÜBERWACHUNG EINER PROZESSGRÖSSE**
DEVICE FOR DETERMINING AND/OR MONITORING A PROCESS QUANTITY
DISPOSITIF POUR DÉTERMINER ET/OU SURVEILLER UNE GRANDEUR DE TRAITEMENT

(30) Priorität: 20.12.2006 DE 102006060921
(43) Veröffentlichungstag der Anmeldung: 26.08.2009
(73) Patentinhaber: Endress+Hauser SE+Co. KG, 79689 Maulburg (DE)
(72) Erfinder: DIETERLE, Roland, 79539 Lörrach (DE); WERNET, Armin, 79618 Rheinfelden (DE)
(74) Vertreter: Andres, Angelika Maria
(86) Internationale Anmeldenummer: PCT/EP2007/063074
(87) Internationale Veröffentlichungsnummer: WO 2008/074611

(56) Entgegenhaltungen:
- EP-A- 1 048 953
- DE-A1- 3 843 339
- US-A- 3 879 644
- US-A- 4 888 989

## Beschreibung

Die Erfindung bezieht sich auf eine Vorrichtung zur Bestimmung und/oder Überwachung mindestens einer Prozessgröße eines Mediums, mit mindestens einer Sondeneinheit, und mit mindestens einer Elektronikeinheit, welche die Sondeneinheit mit einem elektrischen Anregungssignal beaufschlagt und welche von der Sondeneinheit ein elektrisches Messsignal empfängt. Bei dem Medium handelt es sich beispielsweise um eine Flüssigkeit, um ein Schüttgut oder um ein Fluid. Die Prozessgröße ist beispielsweise der Füllstand des Mediums.

In der Prozess- und Automatisierungstechnik ist es bekannt, den Füllstand eines Mediums mittels des kapazitiven Messverfahrens zu bestimmen oder zu überwachen, z.B. wie in DE3843339, US3879644, EP1048953, US4888989. In diesem Verfahren bilden eine Sonde und die Wandung des Behälters bzw. eine zweite Sondeneinheit in Verbindung mit dem Medium als Dielektrium einen Kondensator. Die Kapazität dieses Kondensators wird ausgemessen und ausgehend von diesem Wert wird der Füllstand ermittelt. Eine Problematik des Verfahrens besteht darin, dass die Sondeneinheit mit dem Medium in Kontakt kommt und dass es daher zu einer Ansatzbildung an der Sondeneinheit kommen kann. Ein solcher Ansatz führt dazu, dass die Messung beeinträchtigt wird bzw. dass sie generell verhindert wird. Im Stand der Technik ist es bekannt, die Sondeneinheit mit einer relativ hohen Messfrequenz zu beaufschlagen (z.B. größer 1 MHz), um die Ansatzverträglichkeit zu verbessern. Nachteilig an einer hohen Messfrequenz ist, dass dies mit einer Abnahme der zulässigen maximalen Sondenlänge einhergeht. Dies liegt daran, dass frequenzabhängige Resonanzeffekte auf der Sonde entstehen, welche eine lineare Messung verhindern. Es gilt also einen Kompromiss zwischen einer großen Sondenlänge (z.B. größer 10 m) und einer guten Ansatzverträglichkeit zu finden.

Die Aufgabe der Erfindung besteht darin, ein Messgerät vorzuschlagen, bei welchem zu einer vorgegebenen Sondenlänge eine maximale Ansatzverträglichkeit einstellbar ist.

Die Aufgabe wird erfindungsgemäß dadurch gelöst, dass die Sondeneinheit mindestens eine innere Elektrode und mindestens eine die innere Elektrode umgebende äußere Elektrode aufweist. Die Erfindung besteht somit darin, dass mindestens eine äußere Elektrode um die innere Elektrode angeordnet ist. In einer Ausgestaltung handelt es sich um mindestens zwei äußere Elektroden. Die innere Elektrode und die äußere Elektrode bzw. die äußeren Elektroden sind dabei in einer Ausgestaltung elektrisch unverbunden. In der Ausgestaltung gemäß der Erfindung wird die innere Elektrode oder mindestens eine der äußeren Elektroden mit dem Anregungssignal beaufschlagt. Das Messsignal wird dann ebenfalls von der jeweils mit dem Anregungssignal beaufschlagten Elektrode abgegriffen.

In einer Ausgestaltung sind mindestens zwei äußere Elektroden vorgesehen, welche sich im Wesentlichen in ihrem Abstand zu der inneren Elektrode, welche sie umgeben, voneinander unterscheiden. Entsprechend unterscheiden sich die Elektroden voneinander durch ihren Abstand zur Außenfläche der Sondeneinheit, d.h. die äußeren Elektroden weisen somit jeweils einen anderen Abstand zum Medium auf.

Eine Ausgestaltung beinhaltet, dass die innere Elektrode und die äußere Elektrode gegeneinander und gegenüber dem Medium elektrisch isoliert sind. Die Elektroden sind als jeweils gegeneinander und gegenüber dem Medium durch eine Isolation getrennt.

Eine Ausgestaltung sieht vor, dass die innere Elektrode stabförmig oder seilförmig ausgestaltet ist.

Die Ausgestaltung gemäß der Erfindung beinhaltet, dass die äußere Elektrode zumindest abschnittsweise rohrförmig ausgestaltet ist.

Eine Ausgestaltung sieht vor, dass die Sondeneinheit mindestens zwei äußere Elektroden aufweist, und dass die äußeren Elektroden jeweils die gleiche Wandstärke aufweisen.

Die Ausgestaltung gemäss der Erfindung beinhaltet, dass die äußere Elektrode derartig ausgestaltet ist, dass die äußere Elektrode die innere Elektrode koaxial umgibt. In einer Ausgestaltung ist somit die stabförmige innere Elektrode konzentrisch von mindestens einer äußeren Elektrode bzw. von mehreren äußeren Elektroden umgeben.

Die Ausgestaltung gemäß der Erfindung sieht vor, dass die Elektronikeinheit derartig
ausgestaltet ist, dass die Elektronikeinheit die innere Elektrode oder die äußere Elektrode mit dem Anregungssignal beaufschlagt. In einer Ausgestaltung, in welcher mindestens zwei äußere Elektroden vorgesehen sind, ist die Elektronikeinheit derartig ausgestaltet, dass die Elektronikeinheit die innere Elektrode oder mindestens eine der äußeren Elektroden mit dem Anregungssignal beaufschlagt.

Eine Ausgestaltung beinhaltet, dass die Elektronikeinheit derartig ausgestaltet ist, dass die Elektronikeinheit die innere Elektrode oder die äußere Elektrode in einer vorgebbaren Sequenz mit dem Anregungssignal beaufschlagt. Die äußeren Elektroden werden somit in einer Ausgestaltung zu unterschiedlichen Zeitpunkten mit dem Anregungssignal beaufschlagt.

Eine Ausgestaltung sieht vor, dass mindestens zwei äußere Elektroden vorgesehen sind, und dass die Elektronikeinheit derartig ausgestaltet ist, dass die Elektronikeinheit die innere Elektrode und die äußeren Elektroden abwechselnd mit dem Anregungssignal beaufschlagt. In einer Ausgestaltung wird diese abwechselnde Beaufschlagung bei der Installation des Messgerätes durchgeführt, bei einer anderen Ausgestaltung turnusgemäß und bei einer weiteren Ausgestaltung aufgrund einer manuellen Auslösung. Insbesondere wird auch das Messsignal von der Elektrode abgegriffen, welche mit dem Anregungssignal beaufschlagt wird. Bei der erfindungsgemäßen Sondeneinheit sind somit mehrere Elektroden vorgesehen, welche alle mit dem Anregungssignal beaufschlagbar und von denen das Messsignal empfangbar ist. Die Elektroden sind dabei derartig ausgestaltet und angeordnet, dass der Abstand zwischen Elektrode und Medium jeweils ein anderer ist, d.h. die Isolation zwischen Elektrode und Medium weist jeweils eine unterschiedliche Stärke auf.

Eine Ausgestaltung beinhaltet, dass die Elektronikeinheit derartig ausgestaltet ist, dass die Elektronikeinheit die zu den einzelnen Beaufschlagungen der inneren Elektrode und der äußeren Elektroden mit dem Anregungssignal zugehörigen Messsignale empfängt und auswertet. Indem die einzelnen Elektroden beaufschlagt werden, lässt sich ein Verlauf des Messsignals über die verschiedenen Anregungen bestimmen und die Unterschiede mit Erwartungswerten vergleichen. Durch den Verlauf des Offsets zwischen den einzelnen Anregungen der einzelnen Elektroden, welcher beim Durchscannen ermittelt wird, kann bestimmt werden, mit welcher Elektrode sich das beste Messergebnis erhalten lässt. Ein Kriterium dafür ist, ab wann die Offsetunterschiede nach einer vorgegebenen Näherung in etwa auf einer Gerade liegen, deren Steigung durch die Konstruktion der Sondeneinheit bestimmt und somit bekannt ist. Zum Verständnis kann man das auch so erklären: Scannt man beim Leerabgleich (d.h. das Medium berührt nicht die Sondeneinheit) und Vollabgleich (d.h. die Sondeneinheit wird vollständig vom Medium bedeckt) alle Elektroden durch, müssen bei einem bestimmten Füllstand (z.B. 50%) die aus den Kapazitäten berechneten Füllstände für jede Elektrode auf einer Geraden mit Steigung 0 liegen. Ist das nicht der Fall, ist die Ursache dafür ein Ansatz an der Sondeneinheit, wenn die Abweichung bei der inneren Elektrode liegt bzw. bilden die Ursache Resonanzeffekte, wenn die Abweichung bei der Messung mit den äußeren Elektroden auftritt. In einer Ausgestaltung wird daher die Elektrode für die Messungen verwendet, mit deren Kapazität sich der minimale Füllstand berechnen lässt, wobei die Abweichungen üblicherweise ein positives Vorzeichen haben. Mit der erfindungsgemäßen Sondeneinheit lässt sich somit auch bei der Beaufschlagung der einzelnen Elektroden und der Auswertung der Messsignale beispielsweise ein Ansatz erkennen.

Eine Ausgestaltung sieht vor, dass es sich bei dem Anregungssignal um eine elektrische Wechselspannung handelt.

Eine Ausgestaltung beinhaltet, dass die Elektronikeinheit derartig ausgestaltet ist, dass die Elektronikeinheit das Anregungssignal jeweils mit der gleichen Frequenz oder unterschiedlichen Frequenzen erzeugt.

In einer Ausgestaltung wird das Anregungssignal auf die innere Elektrode (Stab) oder auf eine äußere Elektrode (Rohr) gelegt und die andere Elektrode bzw. in dem Fall, dass es mehr als zwei äußere Elektroden sind, die anderen Elektroden werden jeweils floatend gelassen, d.h. sie sind mit keinem elektrischen Potential verbunden. Je nach Ansteuerung der Elektroden ergeben sich somit unterschiedliche Isolationsstärken zwischen Elektrode und Medium. Dabei wird die Ansatzverträglichkeit mit abnehmender Isolationsdicke besser. Gleichzeitig nimmt die Möglichkeit der linearen Messung mit langen Sondeneinheiten ab. Daher ließe sich anwendungsabhängig umschalten, welche Elektrode zur Messung mit dem Anregungssignal beaufschlagt wird. In einer Ausgestaltung ist dies abhängig von der Länge der Sonde. In einer weiteren Ausgestaltung sind die Dicken der Isolationsschichten zwischen innerer Elektrode und äußeren Elektroden bzw. zwischen den einzelnen äußeren Elektroden unterschiedlich. In einer Ausgestaltung wird das Anregungssignal bei jeder Messung nacheinander auf die einzelnen Elektroden (auf die innere oder eine der äußeren) gegeben und die jeweiligen Messsignale werden getrennt erfasst. Dadurch wird quasi von Messung zu Messung die Isolationsdicke zwischen der beaufschlagten und somit für die Messung aktiven Elektrode und dem Medium reduziert. Zwischen jeder Messung von Elektrode zu Elektrode liegt üblicherweise ein bekannter Offset, der abhängig von der Geometrie ist. Ändert sich der Offset zwischen den Einzelmessungen bei konstanten Schichtdicken, so lässt sich damit auf Ansatz zurückschließen. Durch einen Durchlauf der Messung an den einzelnen äußeren Elektroden kann weiterhin in einer Justierphase bestimmt werden, welches die optimale Isolationsdicke für die vorliegende Anwendung (Länge der Sonde) ist und entsprechend gemessen werden.

Die Erfindung wird anhand der nachfolgenden Zeichnungen näher erläutert. Es zeigt:
Fig. 1: eine schematische Darstellung einer Anwendung eines erfindungsgemäßen Messgerätes, und
Fig. 2: einen Schnitt durch eine erfindungsgemäße Sondeneinheit.

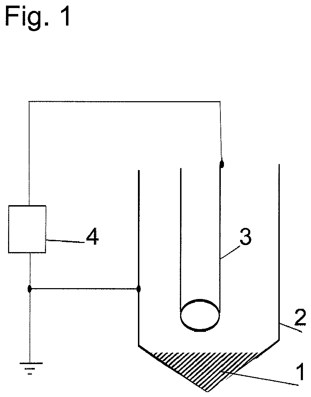

In der Fig. 1 ist prinzipiell das Messverfahren mit dem erfindungsgemäßen Messgerät zur Messung der Prozessgröße Füllstand dargestellt. Das Medium 1, bei welchem es sich beispielsweise um eine Flüssigkeit oder um ein Schüttgut handelt, befindet sich in dem Behälter 2. In dem Behälter weiterhin angebracht ist die Sondeneinheit 3, welche elektrisch mit der Elektronikeinheit 4 verbunden ist. Die Sondeneinheit 3 wird von der Elektronikeinheit 4 mit dem Anregesignal beaufschlagt, bei welchem es sich um ein elektrisches Wechselspannungssignal handelt. Die Sondeneinheit 3 und die Wandung des Behälters 2 bilden mit dem Medium 1 als Dielektrikum einen Kondensator. Dies in dem Fall, dass es sich bei dem Behälter 2 um einen elektrisch leitfähigen Behälter handelt. In einer alternativen, hier nicht dargestellt, Ausgestaltung ist eine zweite Sondeneinheit als zweite Elektrode vorgesehen. Aus dem von der Sondeneinheit 3 abgegriffenen Messsignal lässt sich die Kapazität dieses Kondensators bestimmen. Dessen Kapazität ist dabei abhängig vom Füllstand des Mediums 1 als Dielektrikum.

Das Messsignal ist zunächst ein Stromsignal, welches in den meisten Fällen durch einen Widerstand in ein Spannungssignal umgewandelt wird.

Je nach der Beschaffenheit und Leitfähigkeit des Mediums 1 ist eine Manteleinheit vorgesehen, welche ggf. zumindest teilweise aus Teflon besteht. Diese dient dem Schutz der Sondeneinheit 3 vor dem Medium 1 bzw. verhindert einen elektrischen Kurzschluss zwischen der Sondeneinheit 3 und der Wandung des Behälters 2 bzw. der zweiten, hier nicht dargestellten, Elektrode. In diesem Fall der Manteleinheit bildet nicht das Medium das Dielektrikum des Messkondensators sondern die Isolierung. Der Füllstand wird über die Kapazität des mit leitfähigen Medium abgegriffenen Teils der Sondenisolierung bestimmt. Die Manteleinheit ist somit die Isolation der Elektroden in der Sondeneinheit 3 gegenüber dem Medium 1.

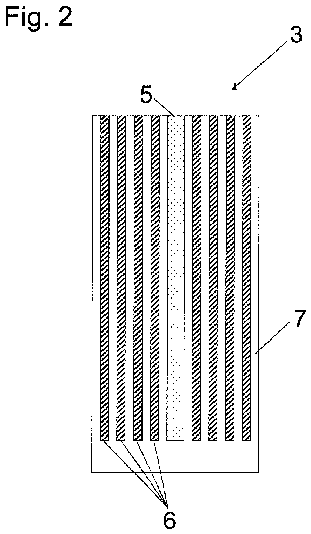

In der Fig. 2 ist ein Schnitt durch eine Ausgestaltung einer erfindungsgemäßen Sondeneinheit 3 dargestellt. Die erfindungsgemäße Sondeneinheit 3 zeigt eine stabförmige innere Elektrode 5 und vier rohrförmige äußere Elektroden 6. Die innere Elektrode 5 ist dabei koaxial von den vier äußeren Elektroden 6 umgeben. Alle Elektroden 5, 6 sind in einer Manteleinheit 7 eingegossen. Diese Manteleinheit 7 ist dabei elektrisch nicht leitfähig, d.h. zwischen der inneren Elektrode 5 und den äußeren Elektroden 6 und zwischen den äußeren Elektroden 6 und dem Medium wirkt sie als Isolationsschicht. Die äußeren Elektroden 6 weisen in der dargestellten Version gleiche Wandstärken auf. In weiteren Ausgestaltungen variieren die einzelnen Wandstärken und die nichtleitfähigen Abschnitte zwischen innerer Elektrode 5 und der von der inneren Elektrode 5 aus ersten äußeren Elektrode 6 bzw. zwischen den äußeren Elektroden 6 untereinander. Je nach der Ausgestaltung der Elektronikeinheit 4 bzw. des in der Elektronikeinheit 4 hinterlegten Programms werden die innere Elektrode 5 und die äußeren Elektroden 6 mit unterschiedlichen Anregungssignalen in unterschiedlichen Abfolgen beaufschlagt. Das Anregesignal ist dabei eine elektrische Wechselspannung. Je nach Ausgestaltung ist dabei die Frequenz des Anregesignals konstant oder sie variiert, z.B. in kontinuierlichen oder diskreten Schritten oder es wird eine Überlagerung mit mehreren Frequenzen auf die Sondeneinheit 3 gegeben.

Die erfindungsgemäße Sondeneinheit 3 ermöglicht somit durch ihren Aufbau eine Veränderung der Isolationsstärke zwischen der für die Messung aktiven Elektrode und dem Medium.

### Bezugszeichenliste

**Tabelle 1**

| | |
|---|---|
| 1 | Medium |
| 2 | Behälter |
| 3 | Sondeneinheit |
| 4 | Elektronikeinheit |
| 5 | Innere Elektrode |
| 6 | Äußere Elektrode |
| 7 | Manteleinheit |

## Patentansprüche

1. Vorrichtung zur kapazitiven Bestimmung und/oder Überwachung mindestens einer Prozessgröße eines Mediums (1),
mit mindestens einer Sondeneinheit (3), welche mindestens eine innere Elektrode (5) aufweist,
und
mit mindestens einer Elektronikeinheit (4), welche die Sondeneinheit (3) mit einem elektrischen Anregungssignal beaufschlagt und welche von der Sondeneinheit ein elektrisches Messsignal empfängt,
**dadurch gekennzeichnet,**
**dass** die Sondeneinheit (3) mindestens eine die innere Elektrode (5) umgebende äußere Elektrode (6) aufweist, wobei die äußere Elektrode (6) zumindest abschnittsweise rohrförmig ausgestaltet ist und wobei die äußere Elektrode (6) derartig ausgestaltet ist, dass sie die innere Elektrode (5) koaxial umgibt,
und **dass** die Elektronikeinheit (4) derartig umschaltbar ausgestaltet ist, dass die Elektronikeinheit (4) anwendungsabhängig die innere Elektrode (5) oder die äußere Elektrode (6) mit dem Anregungssignal beaufschlagt und das Messsignal von der jeweils mit dem Anregungssignal beaufschlagten Elektrode empfängt.

2. Vorrichtung nach Anspruch 1,
**dadurch gekennzeichnet,**
**dass** die innere Elektrode (5) und die äußere Elektrode (6) gegeneinander und gegenüber dem Medium (1) elektrisch isoliert sind.

3. Vorrichtung nach Anspruch 1 oder 2,
**dadurch gekennzeichnet,**
**dass** die innere Elektrode (5) stabförmig oder seilförmig ausgestaltet ist.

4. Vorrichtung nach Anspruch 1 oder 2,
**dadurch gekennzeichnet,**
**dass** die Sondeneinheit (3) mindestens zwei äußere Elektroden (6) aufweist, und
**dass** die äußeren Elektroden (6) jeweils die gleiche Wandstärke aufweisen.

5. Vorrichtung nach Anspruch 1 oder 2,
**dadurch gekennzeichnet,**
**dass** die Elektronikeinheit (4) derartig ausgestaltet ist, dass die Elektronikeinheit (4) die innere Elektrode (5) oder die äußere Elektrode (6) in einer vorgebbaren Sequenz mit dem Anregungssignal beaufschlagt.

6. Vorrichtung nach Anspruch 1, 2 oder 5,
**dadurch gekennzeichnet,**
**dass** mindestens zwei äußere Elektroden (6) vorgesehen sind,
und
**dass** die Elektronikeinheit (4) derartig ausgestaltet ist, dass die Elektronikeinheit (4) die innere Elektrode (5) und die äußeren Elektroden (6) abwechselnd mit dem Anregungssignal beaufschlagt.

7. Vorrichtung nach Anspruch 6,
**dadurch gekennzeichnet,**
**dass** die Elektronikeinheit (4) derartig ausgestaltet ist, dass die Elektronikeinheit (4) die zu den einzelnen Beaufschlagungen der inneren Elektrode (5) und der äußeren Elektroden (6) mit dem Anregungssignal zugehörigen Messsignale empfängt und auswertet.

8. Vorrichtung nach Anspruch 1 oder 2,
**dadurch gekennzeichnet,**
**dass** es sich bei dem Anregungssignal um eine elektrische Wechselspannung handelt.

9. Vorrichtung nach Anspruch 8,
**dadurch gekennzeichnet,**
**dass** die Elektronikeinheit (4) derartig ausgestaltet ist, dass die Elektronikeinheit (4) das Anregungssignal jeweils mit der gleichen Frequenz oder unterschiedlichen Frequenzen erzeugt.

## Claims

1. Apparatus for the capacitance determination and/or monitoring of at last one process variable of a medium (1),
with at least one probe unit (3), which has at least an inner electrode (5), and
with at least one electronics unit (4), which applies an electrical excitation signal to the probe unit (3) and which receives an electrical measuring signal from the probe unit,
**characterized in that**
the probe unit (3) has at least an outer electrode (6) that surrounds the inner electrode (5), wherein the outer electrode (6) is tubular in design at least in part and wherein the outer electrode (6) is designed in such a way that it coaxially surrounds the inner electrode (5),
and **in that** the electronics unit (4) is designed as switchable in such a way that the electronics unit (4) applies the excitation signal to the inner electrode (5) or the outer electrode (6), depending on the application, and receives the measuring signal from the electrode to which the excitation signal is applied.

2. Apparatus as claimed in Claim 1,
**characterized in that**
the inner electrode (5) and the outer electrode (6) are electrically isolated in relation to one another and in relation to the medium (1).

3. Apparatus as claimed in Claim 1 or 2,
**characterized in that**
the inner electrode (5) is designed in the form of a rod or cable.

4. Apparatus as claimed in Claim 1 or 2,
**characterized in that**
the probe unit (3) has at least two outer electrodes (6)
and
**in that** the outer electrodes (6) each has the same wall thickness.

5. Apparatus as claimed in Claim 1 or 2,
**characterized in that**
the electronics unit (4) is designed in such a way that the electronics unit (4) applies the excitation signal to the inner electrode (5) or the outer electrode (6) in a predefined sequence.

6. Apparatus as claimed in Claim 1, 2 or 5,
**characterized in that**
at least two outer electrodes (6) are provided,
and
**in that** the electronics unit (4) is designed in such a way that the electronics unit (4) alternately applies the excitation signal to the inner electrode (5) and the outer electrodes (6).

7. Apparatus as claimed in Claim 6,
**characterized in that**
the electronics unit (4) is designed in such a way that the electronics unit (4) receives and interprets the measuring signals associated with the individual applications of the excitation signal to the inner electrode (5) and the outer electrodes (6).

8. Apparatus as claimed in Claim 1 or 2,
**characterized in that**
the excitation signal is an electrical alternating voltage.

9. Apparatus as claimed in Claim 8,
**characterized in that**
the electronics unit (4) is designed in such a way that the electronics unit (4) generates the excitation signal at the same frequency or different frequencies.

## Revendications

1. Dispositif destiné à la détermination capacitive et/ou la surveillance d'au moins une grandeur process d'un produit (1),
avec au moins une unité de sonde (3), laquelle présente au moins une électrode intérieure (5),
et
avec au moins une unité électronique (4), laquelle unité électronique alimente l'unité de sonde (2) avec un signal d'excitation électrique et laquelle unité électronique reçoit de l'unité de sonde un signal de mesure électrique,
**caractérisé**
**en ce que** l'unité de sonde (3) comprend au moins une électrode extérieure (6) entourant l'électrode intérieure (5), l'électrode extérieure (6) étant conçue au moins partiellement en forme de tube et l'électrode extérieure (6) étant conçue de telle sorte à ce qu'elle entoure de manière coaxiale l'électrode intérieure (5), et en ce que l'unité électronique (4) est conçue de façon commutable afin que l'unité électronique (4) alimente en fonction de l'application l'électrode intérieure (5) ou l'électrode extérieure (6) avec le signal d'excitation, et reçoit le signal de mesure de l'électrode respectivement alimentée avec le signal d'excitation.

2. Dispositif selon la revendication 1,
**caractérisé**
**en ce que** l'électrode intérieure (5) et l'électrode extérieure (6) sont isolées électriquement l'une par rapport à l'autre et par rapport au produit (1).

3. Dispositif selon la revendication 1 ou 2,
**caractérisé**
**en ce que** l'électrode intérieure (5) est conçue en forme de tige ou en forme de câble.

4. Dispositif selon la revendication 1 ou 2,
**caractérisé**
**en ce que** l'unité de sonde (3) comprend au moins deux électrodes extérieures (6) et
**en ce que** les électrodes extérieures (6) présentent chacune la même épaisseur de paroi.

5. Dispositif selon la revendication 1 ou 2,
**caractérisé**
**en ce que** l'unité électronique (4) est conçue de telle sorte que l'unité électronique (4) alimente selon une séquence prédéfinissable l'électrode intérieure (5) ou l'électrode extérieure (6) avec le signal d'excitation.

6. Dispositif selon la revendication 1, 2 ou 5,
**caractérisé**
**en ce que** sont prévues au moins deux électrodes extérieures (6),
et
**en ce que** l'unité électronique (4) est conçue de telle sorte que l'unité électronique (4) alimente alternativement l'électrode intérieure (5) et les électrodes extérieures (6) avec le signal d'excitation.

7. Dispositif selon la revendication 6,
**caractérisé**
**en ce que** l'unité électronique (4) est conçue de telle sorte que l'unité électronique (4) reçoit et interprète les signaux de mesure correspondants aux différentes alimentations de l'électrode intérieure (5) et des électrodes extérieures (6) avec le signal d'excitation.

8. Dispositif selon la revendication 1 ou 2,
**caractérisé**
**en ce que**, concernant le signal d'excitation, il s'agit d'une tension alternative électrique.

9. Dispositif selon la revendication 8,
**caractérisé**
**en ce que** l'unité électronique (4) est conçue de telle sorte que l'unité électronique (4) génère le signal d'excitation respectivement avec la même fréquence ou avec des fréquences différentes.
